# EUROPEAN PATENT APPLICATION

(11) **EP 1 844 787 A1**
(43) Date of publication of application: **17.10.2007**
(21) Application number: 06711797.8
(22) Date of filing: 17.01.2006
(51) Int. Cl.: A61K 38/00, A61K 45/00, A61P 27/02

(54) **DRUGS FOR NERVE CELL REGENERATION**

(30) Priority: 18.01.2005 JP 2005010189
(71) Applicant: Kyoto University, Kyoto-shi, Kyoto 606-8501 (JP)
(72) Inventor: TAKAHASHI, Masayo c/o Kyoto University Hospital, Kyoto-shi, Kyoto, 606-8507 (JP); MANDAI, Michiko c/o Kyoto University Hospital, Kyoto-shi, Kyoto, 606-8507 (JP); OOTO, Sotaro c/o Graduate School of Medicine, Kyoto Univ., Kyoto 606-8501 (JP); OSAKADA, Fumikata c/o Grad. School of Pharmac. Sciences, Kyoto-shi, Kyoto 606-8501 (JP)
(74) Representative: Gray, James
(86) International application number: PCT/JP2006/300517
(87) International publication number: WO 2006/077824

(57) **Abstract**

Wnt3a proteins represented by SEQ ID NO:2, etc. are used as the active ingredient of remedy for nerve cell regeneration, in particular, remedy for retinal cell regeneration which is useful in treating retinitis pigmentosa, age-related macular degeneration, cone dystrophy and so on.

## Description

### Technical Field

The present invention relates to a remedy for nerve cell regeneration.

### Background Art

Heretofore, nerve cells such as retinal cells of living mammals have been considered that they cannot regenerate. Recently, it has been reported that Muller cells can acquire properties like those of precursor cells by division in response to an acute disorder, and some of which differentiate into retinal neurons. However, the number of Muller cells to be activated was very small (Non-Patent Document 1).

Wnt is a secretory glycoprotein, or a signaling factor essential to body axis formation and formation of the central nerve system and limbs. In recent years, Wnt3a, one of the Wnt family members, has been reported to be associated with self-duplication of hematopoietic stem cells (Non-Patent Document 2). In addition, Wnt2b has been reported to contribute to proliferation of precursor cells of the ciliary marginal zone and maintenance of undifferentiated status thereof in the chick fetal retina (Non-Patent Document 3).
Further, Wnt-6 has been reported to be therapeutically useful in neurogenic disorders, carcinomas, cardiovascular disorders, strokes, developmental disabilities, and so on (Patent Document 1). Further, Wnt-10a has been reported to be therapeutically useful in neurogenic disorders (Patent Document 2). Besides, a method of enhancing proliferation, differentiation, or maintenance of haematopoietic stem cells / primordial cells with Wnt has also been reported (Patent Document 3). However, involvement of Wnt in neuronal regeneration has not been known in the art.
[Patent Document 1] JP 2000-060575 A
[Patent Document 2] WO 99/38966
[Patent Document 3] WO 98/06747
[Non Patent Document 1] Proc Natl Acad Sci USA. 2004 Sep 14; 101(37): 13654-9
[Non Patent Document 2] Nature 423, 409-414(2003)
[Non Patent Document 3] Development 130, 587-598(2003)

### Disclosure of the Invention

An object of the present invention is to provide a remedy to be used for nerve cell regeneration.

The inventors of the present invention have intensively studied for addressing the aforementioned object. As a result, the inventors of the present invention have found that a Wnt3a protein or a Wnt3a signaling activator has an effect of inducing Muller cells to proliferate to and differentiate into retinal cells. Therefore, they have found that neural cells such as retinal cells can regenerate by using the Wnt3a protein, and thus the present invention has been completed.

That is, the present invention provides:
(1) A remedy for nerve cell regeneration, comprising a Wnt3a protein or a Wnt3a signaling activator.
(2) The remedy according to (1), wherein the nerve cell is retinal cell.
(3) The remedy according to (2), wherein the remedy is a therapeutic drug for retinitis pigmentosa, age-related macular degeneration, or cone dystrophy.
(4) The remedy according to any one of (1) to (3), wherein the Wnt3a protein has a sequence of amino acids 25 to 352 in SEQ ID No. 2 or a sequence of amino acids Nos. 25 to 352 in SEQ ID No. 2 with substitution, deletion, insertion, or addition of one or several amino acids and has an ability of regenerating nerve cells.

### Brief Description of Drawings

Fig. 1 is a diagram (photographs) that shows the results of immunostaining with a BrdU antibody which was performed on retinal sections added with control or Wnt3a. INL denotes the inner nuclear layer and ONL denotes the neuroepithelial layer of retina.
Fig. 2 is a graph that shows the percentages of BRdU-positive cells in the retina which was added with control or Wnt3a. GCL (left) denotes the ganglionic cell layer and INL (middle) denotes the inner nuclear layer, and ONL (right) denotes the neuroepithelial layer of retina.
Fig. 3 is a diagram (photograph) that represents the results of immunostaining with a glutamine synthetase (GS) antibody and a BrdU antibody. The arrows indicate portions where both GS and BrdU are positive. GCL denotes the ganglionic cell layer. INL denotes the inner nuclear layer and ONL denotes the neuroepithelial layer of retina.
Fig. 4 is a diagram (photograph) that represents a merged image of the results of immunostaining with glutamine synthetase antibody and BrdU antibody which was performed on a retinal section added with an inhibitor of glycogen synthase kinase 3β. Cells brightly-stained in the middle of the section are BrdU-positive cells.

### Description of the preferred embodiments

The remedy of the present invention comprises a Wnt3a protein as an active ingredient. The Wnt3a protein is preferably a human or mouse Wnt3a protein, and particularly preferably a human Wnt3a protein. An example of a human Wnt3a protein includes a protein having an amino acid sequence of SEQ ID No. 2. An example of a mouse Wnt3a protein includes a protein having an amino acid sequence of SEQ ID No. 4. The Wnt3a protein may be contained in the remedy of the present invention as a full-length protein (precursor protein) or may be preferably contained in the remedy of the present invention as a mature protein which is generated by cleavage of a signal sequence. The human mature Wnt3a protein may be a protein having an amino acid sequence of amino acids 25 to 352 in SEQ ID No. 2. The mouse mature Wnt3a protein may be a protein having an amino acid sequence of amino acids 25 to 352 in SEQ ID No. 4.

Further, it is easily expected that Wnt3a proteins have sequence variations due to amino acid substitutions or the like depending on their respective biological species in origin. In addition, the ability of the Wnt3a protein to regenerate nerve cell is considered to be not affected even by replacement of an amino acid with another amino acid having analogous properties (i.e., conservative substitution) or deletion of an amino acid at a portion not involved in the activity. As long as the nerve cell regeneration ability is retained, the Wnt3a protein may have an amino acid sequence in which the substitution, deletion, addition, or insertion of one or several amino acids has occurred in the amino acid sequence of SEQ ID No. 2 or 4 or in the amino acid sequence of amino acids 25 to 352 in SEQ ID No. 2 or in the amino acid sequence of amino acids 25 to 352 in SEQ ID No. 4. In this case, the term "several" means preferably 2 to 20, more preferably 2 to 10, and particularly preferably 2 to 5. Also, a protein having high homology, such as not less than 90%, and more preferably not less than 95% homology to the amino acid sequences as described above may be used as long as the protein retains the nerve cell regeneration ability.

The Wnt3a protein may be one purified from a biological source or one chemically synthesized, and preferably one obtained by gene recombination. Further, any Wnt3a protein commercially available may be used.
For obtaining the Wnt3a protein by gene recombination, for example, a DNA having a nucleotide sequence of SEQ ID No. 1 (human wnt3a gene) or SEQ ID No. 3 (mouse wnt 3a gene) is introduced into *E. coli* cells, animal cells, a non-human transgenic animal or the like to express a recombinant protein, followed by purification of the protein. In addition, the protein may not always be a purified one. A partially purified product or cell extract may be used for the detection of interaction. Examples of vectors for introducing the above-mentioned DNA into *E. coli* include pET vector (Novagen Co., Ltd.) and pGEX vector (Amersham Pharmacia Co., Ltd.), while an example of a vector for introducing the DNA into animal cells includes pcDNA vector (Invitrogen Corporation).

The Wnt3a protein can be used for manufacturing a remedy for nerve cell regeneration. The Wnt3a proteins obtained as described above may be directly used as the remedy of the present invention. Alternatively, a remedy may be obtained by combining a pharmaceutically acceptable carrier with the Wnt3a protein. Examples of the carriers include vehicles, stabilizers, tonicity adjusting agents, and buffers.
The formulations of the remedy of the present invention are not specifically limited. For example, formulations for oral administration specifically include tablets, pills, encapsulated agents, granular agents, syrups, emulsions, and suspension agents.
Formulations for parenteral administrations include ointments, creams, injections, fomentations, liniments, suppositories, eye-dropper, nasal absorbents, trans-pulmonary absorbents, and transdermal absorbents.
In particular, ophthalmological local applications preferably include injections (such as systematic application, intravitreal administration, subretinal application, Tenon's capsule application, and subconjunctival administration), trans keratoconjunctival agents, eye-droppers, and ophthalmic ointments.
Any formulation in solution can be prepared by any conventional method, for example, the Wnt3a protein is usually dissolved in an axenic aqueous solution to be used for injection, suspended in an extract, or emulsified and embed in riposome. The formulation may be prepared by combining any of tonicity adjusting agents (such as sodium chloride, potassium chloride, boric acid, or glycerin), buffers (such as a boric acid buffer solution, a phosphate buffer, or an acetate buffer), solubilizing agents (such as surfactants or cyclodextrin), stabilizers (such as citric acid, ethylene diamine tetraacetic acid, sodium sulfite, or sodium bisulfite), and viscosity-imparting agents (such as synthetic polymers, celluloses, or polyalcohols). In addition, for stabilizing the Wnt3a protein, any additional protein such as human serum albumin may be combined.
When the remedy of the present invention is formulated as eye droppers, any of tonicity adjusting agents such as sodium chloride and concentrated glycerin, buffers such as sodium phosphate and sodium acetate, surfactants such as polyoxyethylene sorbitan monoolate, polyoxyl 40 stearate, and polyoxyethylene hydrogenated castor oil, stabilizers such as sodium citrate and sodium edetate, and preservatives such as benzalkonium chloride and paraben may be used if desired. The remedy may be of any pH as long as it is within a range permitted by ophthalmological formulation, and preferably pH of 4 to 8.

The Wnt3a protein has an effect of regenerating nerve cells such as retinal cells. Thus, it can be widely applied as therapeutic agents for diseases that causes neurodegeneration and neural function disorders. The regeneration of nerve cells can be confirmed using as an index the ability to accelerate the differentiation from precursor cells to nerve cells, for example, based on the expression amount of a marker gene specific to nerve cells at the time of addition of Wnt3a to the precursor cells. For example, the retinal cell regeneration effect (differentiation-accelerating effect) can be confirmed by investigating an increase in cell division of Muller cells or an increase in retinal cells using staining with a Rhodopsin antibody or the like. The Wnt3a protein contained in the remedy of the present invention preferably increases the division of Muller cells not less than 10%, and more preferably not less than 50% as compared to the untreated state.

When the remedy of the present invention is applied for the regeneration of retinal cells, the remedy can be applied as a therapeutic remedy for visual function improvement to the ophthalmology disorder that causes retinal degeneration and visual function disorder. Example of the ophthalmology disorder that causes retinal degeneration and visual function disorder include, in addition to those caused by inflammation or trauma, glaucoma (glaucoma primarium, secondary glaucoma), retinal vascular occlusion, diabetic retinopathy, ischemic optic neuropathy, macular degeneration, retinitis pigmentosa, Leber's hereditary optic neuropathy, Oguchi's disease, angioid streaks, retinal periphlebitis, Eales disease, ischemic eye syndrome, retinal arteriolar macroaneurysm, retinopathy by hypertension, renal disease, or the hemopathy, retinal dystrophy, macular dystrophy, macular oedema, denatured retinoschisis, and cone dystrophy.

The remedy of the present invention is systemically or locally administered. The effective dosage and the number of doses vary depending on dosage formulation, administration route, or the age, weight, disorder to be treated, symptom, or severity of the patient. Generally, 0.001 to 100 mg, and preferably 0.01 to 10 mg per an adult can be administered in a single dose or several divided doses.

On the other hand, a compound that enhances the activity of Wnt3a signaling or a compound that activates the transcription of Wnt3a (hereinafter, these compounds will be referred to as Wnt3a signaling activator) can be used as effective ingredient in the remedy for nerve cell regeneration. The Wnt3a signaling activator may be an inhibitor of glycogen synthase kinase 3β (GSK-3β), which has been known to be associated with the signal pathway of Wnt3a (EMBO J. 2005 Apr 20; 24(8): 1571-83.). Examples of a GSK-3β inhibitor include any of known GSK-3β inhibitors or a compound obtained by screening using GSK-3β activity as an index. The GSK-3β inhibitor may be, for example, AR-A014418 (J. Biol., Chem., Vol.278, No. 46, p45937-45945, 2003). Besides, a physiologically acceptable salt, hydrate, or solvate of such a compound or an analog thereof may be also used. The remedy comprising the GSK-3β inhibitor as an effective ingredient can be formulated by an ordinary method. The applicable disorders include those described above.

### Examples

Hereinafter, the present invention will be described in more detail by way of examples. However, the invention will not be restricted by these examples.

The retina excluding the pigment epithelium was obtained from a 6-week-old adult DA rat (obtained from Shimizu Laboratory Supplies Co., Ltd. (Kyoto)) and a retinal organ culture was then carried out on a Millicell chamber filter. The retinal organ culture was conducted according to a method described in Methods2002; 28: 387-395 and Brain Res 2002; 954: 286-293. The culture medium used was 50% Minimum Essential Medium + HEPES (Invitrogen Corporation) / 25% Hank's solution (Invitrogen Corporation) / 25% inactivated horse serum / 200 µM L-glutamine / 5.75 mg/ml glucose.
Wnt3a (100 ng/ml; R&D Systems) and BrdU (5ng/ml; SIGMA) were added to the culture medium everyday and a frozen specimen was then prepared after 4 days, followed by investigating the presence or absence of BrdU-positive divided cells by an immunological staining with a BrdU antibody.

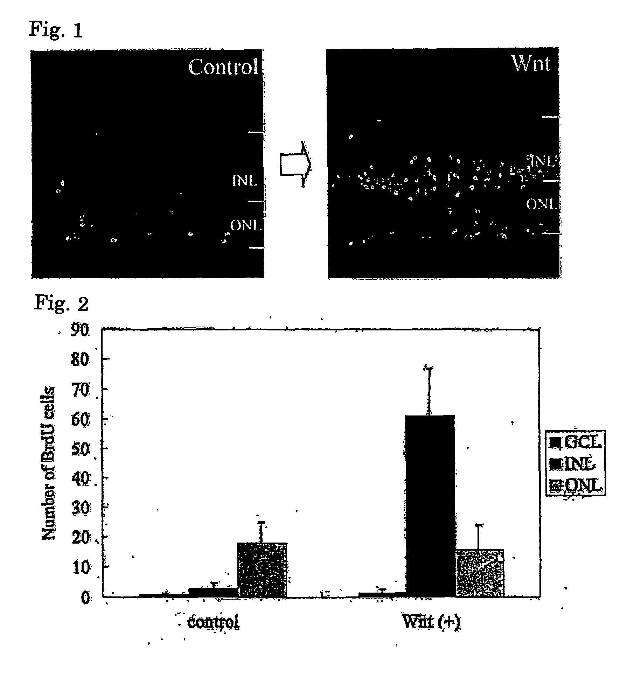

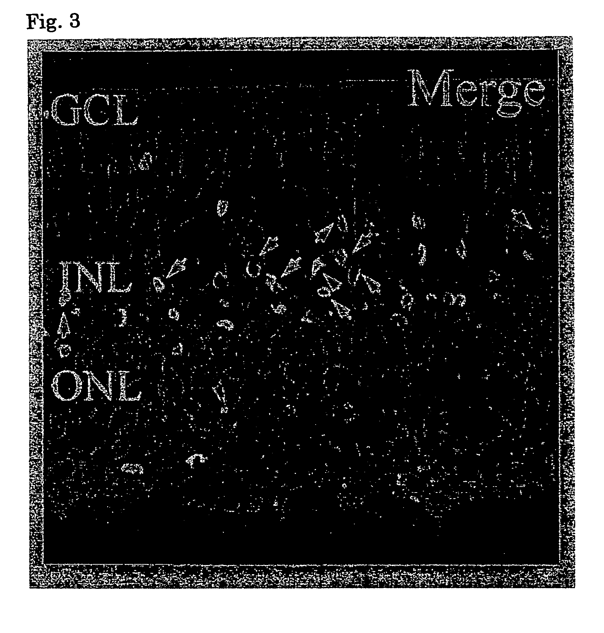

The results are shown in Fig. 1. Compared with the control which did not contain Wnt3a, organ culture added with Wnt3a showed many BrdU-positive divided cells in the retinal inner nuclear layer (INL) (Fig. 1). The results obtained by the calculation of the ratio of the divided cells are shown in Fig. 2. As is evident from the figure, the addition of Wnt3a caused a dramatic increase in the number of the divided cells in INL. As investigated by an immunological staining, most of these divided cells were identified as glutamine synthetase positive Muller cells (Fig. 3). As is evident from these results, the division of the Muller cells has been accelerated by Wnt3a.

Further, after 4 day culture with the addition of Wnt 3a, the culture medium was changed, and the culture was then cultured for 1 week without Wnt 3a. As a result, a part of the divided cells express Rhodopsin as a marker of the visual cells, which showed that the Muller cells differentiated into retinal nerve cells.
In this case, an effect of accelerating the differentiation to retinal cells was shown, but it was suggested that Wnt 3a also shows an effect of accelerating the differentiation of other neural cells. Wnt3a was expected to have a regeneration effect on a wide variety of neural cells.

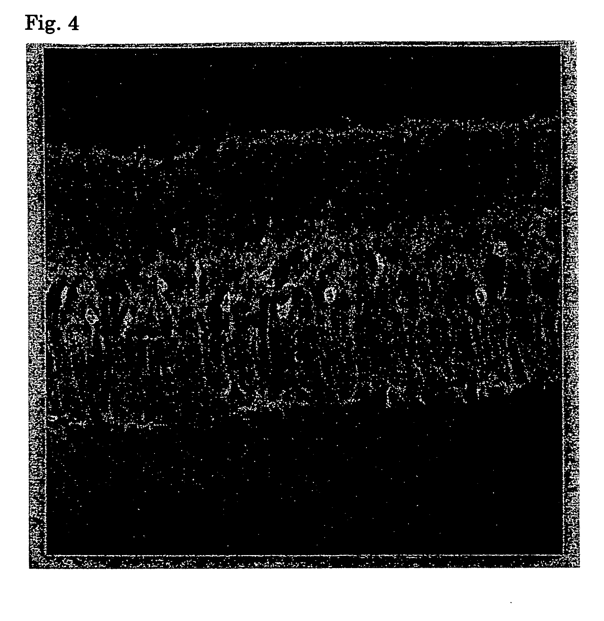

Further, an inhibitor of glycogen synthase kinase 3β(GSK-3β), AR-A014418(J, Biol. Chem., Vol, 278, No. 46, p45937-45945, 2003), was added to a culture medium for the retina organ culture and the effect thereof was examined. After incubating for 4 days with the addition of 5 µM AR-A014418 together with BrdU, as shown in Fig. 4, the number of BrdU-positive cells was increased. In contrast, the number of BrdU-positive cells in control without the addition ofAR-AO14418 was small. From this fact, like the Wnt3a protein, the possibility that the GSK-3β inhibitor could be provided as a remedy for regeneration of nerve cells such as retinal cells was suggested.

### Industrial Applicability

The remedy of the present invention can accelerate the regeneration of nerve cells and can be used to treat the disorders such as neurodegeneration disorder or neurologic dysfunction. In particular, the remedy of the present invention can be used to treat any disorder such as the retinitis pigmentosa, in which visual cells are irreversibly denatured, by regenerating the visual cells in the retina through activation of endogenous stem cells.

## Claims

1. A remedy for nerve cell regeneration, comprising a Wnt3a protein or a Wnt3a signaling activator.

2. The remedy according to claim 1, wherein the nerve cell is retinal cell.

3. The remedy according to claim 2, wherein the remedy is a therapeutic drug for retinitis pigmentosa, age-related macular degeneration, or cone dystrophy.

4. The remedy according to any one of claims 1 to 3, wherein the Wnt3a protein has a sequence of amino acids 25 to 352 in SEQ ID No. 2 or a sequence of amino acids Nos. 25 to 352 in SEQ ID No. 2 with substitution, deletion, insertion, or addition of one or several amino acids and has an ability of regenerating nerve cells.


SEQUENCE LISTING

<110>  Kyoto University

<120>  REMEDY FOR NERVE CELL REGENERATION

<130>  OP-C5292-PCT

<150>  JP2005-10189
<151>  2005-01-18

<160>  4

<170>  PatentIn version 3.1

<210>  1
<211>  1059
<212>  DNA
<213>  Homo sapiens

<220>
<221>  CDS
<222>  (1)..(1056)

<400>  1
atg gcc cca ctc gga tac ttc tta ctc ctc tgc agc ctg aag cag gct      48
Met Ala Pro Leu Gly Tyr Phe Leu Leu Leu Cys Ser Leu Lys Gln Ala
1               5                   10                  15

ctg ggc agc tac ccg atc tgg tgg tcg ctg gct gtt ggg cca cag tat      96
Leu Gly Ser Tyr Pro Ile Trp Trp Ser Leu Ala Val Gly Pro Gln Tyr
            20                  25                  30

tcc tcc ctg ggc tcg cag ccc atc ctg tgt gcc agc atc ccg ggc ctg      144
Ser Ser Leu Gly Ser Gln Pro Ile Leu Cys Ala Ser Ile Pro Gly Leu
        35                  40                  45

gtc ccc aag cag ctc cgc ttc tgc agg aac tac gtg gag atc atg ccc      192
Val Pro Lys Gln Leu Arg Phe Cys Arg Asn Tyr Val Glu Ile Met Pro
    50                  55                  60

agc gtg gcc gag ggc atc aag att ggc atc cag gag tgc cag cac cag      240
Ser Val Ala Glu Gly Ile Lys Ile Gly Ile Gln Glu Cys Gln His Gln
65                  70                  75                  80

ttc cgc ggc cgc cgg tgg aac tgc acc acc gtc cac gac agc ctg gcc      288
Phe Arg Gly Arg Arg Trp Asn Cys Thr Thr Val His Asp Ser Leu Ala
                85                  90                  95

atc ttc ggg ccc gtg ctg gac aaa gct acc agg gag tcg gcc ttt gtc      336
Ile Phe Gly Pro Val Leu Asp Lys Ala Thr Arg Glu Ser Ala Phe Val

            100                 105                 110
cac gcc att gcc tca gcc ggt gtg gcc ttt gca gtg aca cgc tca tgt 384
His Ala Ile Ala Ser Ala Gly Val Ala Phe Ala Val Thr Arg Ser Cys
        115                 120                 125
gca gaa ggc acg gcc gcc atc tgt ggc tgc agc agc cgc cac cag ggc 432
Ala Glu Gly Thr Ala Ala Ile Cys Gly Cys Ser Ser Arg His Gln Gly
    130                 135                 140
tca cca ggc aag ggc tgg aag tgg ggt ggc tgt agc gag gac atc gag 480
Ser Pro Gly Lys Gly Trp Lys Trp Gly Gly Cys Ser Glu Asp Ile Glu
145                 150                 155                 160
ttt ggt ggg atg gtg tct cgg gag ttc gcc gac gcc cgg gag aac cgg 528
Phe Gly Gly Met Val Ser Arg Glu Phe Ala Asp Ala Arg Glu Asn Arg
                165                 170                 175
cca gat gcc cgc tca gcc atg aac cgc cac aac aac gag gct ggg cgc 576
Pro Asp Ala Arg Ser Ala Met Asn Arg His Asn Asn Glu Ala Gly Arg
            180                 185                 190
cag gcc atc gcc agc cac atg cac ctc aag tgc aag tgc cac ggg ctg 624
Gln Ala Ile Ala Ser His Met His Leu Lys Cys Lys Cys His Gly Leu
        195                 200                 205
tcg ggc agc tgc gag gtg aag aca tgc tgg tgg tcg caa ccc gac ttc 672
Ser Gly Ser Cys Glu Val Lys Thr Cys Trp Trp Ser Gln Pro Asp Phe
    210                 215                 220
cgc gcc atc ggt gac ttc ctc aag gac aag tac gac agc gcc tcg gag 720
Arg Ala Ile Gly Asp Phe Leu Lys Asp Lys Tyr Asp Ser Ala Ser Glu
225                 230                 235                 240
atg gtg gtg gag aag cac cgg gag tcc cgc ggc tgg gtg gag acc ctg 768
Met Val Val Glu Lys His Arg Glu Ser Arg Gly Trp Val Glu Thr Leu
                245                 250                 255
cgg ccg cgc tac acc tac ttc aag gtg ccc acg gag cgc gac ctg gtc 816
Arg Pro Arg Tyr Thr Tyr Phe Lys Val Pro Thr Glu Arg Asp Leu Val
            260                 265                 270
tac tac gag gcc tcg ccc aac ttc tgc gag ccc aac cct gag acg ggc 864
Tyr Tyr Glu Ala Ser Pro Asn Phe Cys Glu Pro Asn Pro Glu Thr Gly
        275                 280                 285
tcc ttc ggc acg cgc gac cgc acc tgc aac gtc agc tcg cac ggc atc 912
Ser Phe Gly Thr Arg Asp Arg Thr Cys Asn Val Ser Ser His Gly Ile
    290                 295                 300
gac ggc tgc gac ctg ctg tgc tgc ggc cgc ggc cac aac gcg cga gcg 960
Asp Gly Cys Asp Leu Leu Cys Cys Gly Arg Gly His Asn Ala Arg Ala
305                 310                 315                 320
gag cgg cgc cgg gag aag tgc cgc tgc gtg ttc cac tgg tgc tgc tac 1008
Glu Arg Arg Arg Glu Lys Cys Arg Cys Val Phe His Trp Cys Cys Tyr
                325                 330                 335
gtc agc tgc cag gag tgc acg cgc gtc tac gac gtg cac acc tgc aag 1056
Val Ser Cys Gln Glu Cys Thr Arg Val Tyr Asp Val His Thr Cys Lys

340                 345                 350
tag                                                                    1059

<210>  2
<211>  352
<212>  PRT
<213>  Homo sapiens

<400>  2
Met Ala Pro Leu Gly Tyr Phe Leu Leu Leu Cys Ser Leu Lys Gln Ala
1               5                   10                  15
Leu Gly Ser Tyr Pro Ile Trp Trp Ser Leu Ala Val Gly Pro Gln Tyr
            20                  25                  30
Ser Ser Leu Gly Ser Gln Pro Ile Leu Cys Ala Ser Ile Pro Gly Leu
        35                  40                  45
Val Pro Lys Gln Leu Arg Phe Cys Arg Asn Tyr Val Glu Ile Met Pro
    50                  55                  60
Ser Val Ala Glu Gly Ile Lys Ile Gly Ile Gln Glu Cys Gln His Gln
65                  70                  75                  80
Phe Arg Gly Arg Arg Trp Asn Cys Thr Thr Val His Asp Ser Leu Ala
                85                  90                  95
Ile Phe Gly Pro Val Leu Asp Lys Ala Thr Arg Glu Ser Ala Phe Val
            100                 105                 110
His Ala Ile Ala Ser Ala Gly Val Ala Phe Ala Val Thr Arg Ser Cys
        115                 120                 125
Ala Glu Gly Thr Ala Ala Ile Cys Gly Cys Ser Ser Arg His Gln Gly
    130                 135                 140
Ser Pro Gly Lys Gly Trp Lys Trp Gly Gly Cys Ser Glu Asp Ile Glu
145                 150                 155                 160
Phe Gly Gly Met Val Ser Arg Glu Phe Ala Asp Ala Arg Glu Asn Arg
                165                 170                 175
Pro Asp Ala Arg Ser Ala Met Asn Arg His Asn Asn Glu Ala Gly Arg
            180                 185                 190
Gln Ala Ile Ala Ser His Met His Leu Lys Cys Lys Cys His Gly Leu
        195                 200                 205
Ser Gly Ser Cys Glu Val Lys Thr Cys Trp Trp Ser Gln Pro Asp Phe
    210                 215                 220
Arg Ala Ile Gly Asp Phe Leu Lys Asp Lys Tyr Asp Ser Ala Ser Glu
225                 230                 235                 240
Met Val Val Glu Lys His Arg Glu Ser Arg Gly Trp Val Glu Thr Leu
                245                 250                 255
Arg Pro Arg Tyr Thr Tyr Phe Lys Val Pro Thr Glu Arg Asp Leu Val
            260                 265                 270
Tyr Tyr Glu Ala Ser Pro Asn Phe Cys Glu Pro Asn Pro Glu Thr Gly

Ser Phe Gly Thr Arg Asp Arg Thr Cys Asn Val Ser Ser His Gly Ile
    290                 295                 300
Asp Gly Cys Asp Leu Leu Cys Cys Gly Arg Gly His Asn Ala Arg Ala
305                 310                 315                 320
Glu Arg Arg Arg Glu Lys Cys Arg Cys Val Phe His Trp Cys Cys Tyr
                325                 330                 335
Val Ser Cys Gln Glu Cys Thr Arg Val Tyr Asp Val His Thr Cys Lys
            340                 345                 350

<210>  3
<211>  1059
<212>  DNA
<213>  Mus musculus

<220>
<221>  CDS
<222>  (1)..(1056)

<400>  3
atg gct cct ctc gga tac ctc tta gtg ctc tgc agc ctg aag cag gct      48
Met Ala Pro Leu Gly Tyr Leu Leu Val Leu Cys Ser Leu Lys Gln Ala
1               5                   10                  15
ctg ggc agc tac ccg atc tgg tgg tcc ttg gct gtg gga ccc cag tac      96
Leu Gly Ser Tyr Pro Ile Trp Trp Ser Leu Ala Val Gly Pro Gln Tyr
            20                  25                  30
tcc tct ctg agc act cag ccc att ctc tgt gcc agc atc cca ggc ctg      144
Ser Ser Leu Ser Thr Gln Pro Ile Leu Cys Ala Ser Ile Pro Gly Leu
        35                  40                  45
gta ccg aag cag ctg cgc ttc tgc agg aac tac gtg gag atc atg ccc      192
Val Pro Lys Gln Leu Arg Phe Cys Arg Asn Tyr Val Glu Ile Met Pro
    50                  55                  60
agc gtg gct gag ggt gtc aaa gcg ggc atc cag gag tgc cag cac cag      240
Ser Val Ala Glu Gly Val Lys Ala Gly Ile Gln Glu Cys Gln His Gln
65                  70                  75                  80
ttc cga ggc cgg cgt tgg aac tgc acc acc gtc agc aac agc ctg gcc      288
Phe Arg Gly Arg Arg Trp Asn Cys Thr Thr Val Ser Asn Ser Leu Ala
                85                  90                  95
atc ttt ggc cct gtt ctg gac aaa gcc acc cgg gag tca gcc ttt gtc      336
Ile Phe Gly Pro Val Leu Asp Lys Ala Thr Arg Glu Ser Ala Phe Val
            100                 105                 110
cat gcc atc gcc tcc gct gga gta gct ttc gca gtg aca cgc tcc tgt      384
His Ala Ile Ala Ser Ala Gly Val Ala Phe Ala Val Thr Arg Ser Cys
        115                 120                 125

gca gag gga tca gct gct atc tgt ggg tgc agc agc cgc ctc cag ggc 432
Ala Glu Gly Ser Ala Ala Ile Cys Gly Cys Ser Ser Arg Leu Gln Gly
    130                 135                 140
tcc cca ggc gag ggc tgg aag tgg ggc ggc tgt agt gag gac att gaa 480
Ser Pro Gly Glu Gly Trp Lys Trp Gly Gly Cys Ser Glu Asp Ile Glu
145                 150                 155                 160
ttt gga gga atg gtc tct cgg gag ttt gcc gat gcc agg gag aac cgg 528
Phe Gly Gly Met Val Ser Arg Glu Phe Ala Asp Ala Arg Glu Asn Arg
                165                 170                 175
ccg gat gcc cgc tct gcc atg aac cgt cac aac aat gag gct ggg cgc 576
Pro Asp Ala Arg Ser Ala Met Asn Arg His Asn Asn Glu Ala Gly Arg
            180                 185                 190
cag gcc atc gcc agt cac atg cac ctc aag tgc aaa tgc cac ggg cta 624
Gln Ala Ile Ala Ser His Met His Leu Lys Cys Lys Cys His Gly Leu
        195                 200                 205
tct ggc agc tgt gaa gtg aag acc tgc tgg tgg tcg cag ccg gac ttc 672
Ser Gly Ser Cys Glu Val Lys Thr Cys Trp Trp Ser Gln Pro Asp Phe
    210                 215                 220
cgc acc atc ggg gat ttc ctc aag gac aag tat gac agt gcc tcg gag 720
Arg Thr Ile Gly Asp Phe Leu Lys Asp Lys Tyr Asp Ser Ala Ser Glu
225                 230                 235                 240
atg gtg gta gag aaa cac cga gag tct cgt ggc tgg gtg gag acc ctg 768
Met Val Val Glu Lys His Arg Glu Ser Arg Gly Trp Val Glu Thr Leu
                245                 250                 255
agg cca cgt tac acg tac ttc aag gtg ccg aca gaa cgc gac ctg gtc 816
Arg Pro Arg Tyr Thr Tyr Phe Lys Val Pro Thr Glu Arg Asp Leu Val
            260                 265                 270
tac tac gag gcc tca ccc aac ttc tgc gaa cct aac ccc gaa acc ggc 864
Tyr Tyr Glu Ala Ser Pro Asn Phe Cys Glu Pro Asn Pro Glu Thr Gly
        275                 280                 285
tcc ttc ggg acg cgt gac cgc acc tgc aat gtg agc tcg cat ggc ata 912
Ser Phe Gly Thr Arg Asp Arg Thr Cys Asn Val Ser Ser His Gly Ile
    290                 295                 300
gat ggg tgc gac ctg ttg tgc tgc ggg cgc ggg cat aac gcg cgc act 960
Asp Gly Cys Asp Leu Leu Cys Cys Gly Arg Gly His Asn Ala Arg Thr
305                 310                 315                 320
gag cga cgg agg gag aaa tgc cac tgt gtt ttc cat tgg tgc tgc tac 1008
Glu Arg Arg Arg Glu Lys Cys His Cys Val Phe His Trp Cys Cys Tyr
                325                 330                 335
gtc agc tgc cag gag tgc aca cgt gtc tat gac gtg cac acc tgc aag 1056
Val Ser Cys Gln Glu Cys Thr Arg Val Tyr Asp Val His Thr Cys Lys
            340                 345                 350
tag 1059

<210> 4
<211> 352
<212> PRT
<213> Mus musculus

<400> 4
Met Ala Pro Leu Gly Tyr Leu Leu Val Leu Cys Ser Leu Lys Gln Ala
1               5                   10                  15
Leu Gly Ser Tyr Pro Ile Trp Trp Ser Leu Ala Val Gly Pro Gln Tyr
            20                  25                  30
Ser Ser Leu Ser Thr Gln Pro Ile Leu Cys Ala Ser Ile Pro Gly Leu
        35                  40                  45
Val Pro Lys Gln Leu Arg Phe Cys Arg Asn Tyr Val Glu Ile Met Pro
    50                  55                  60
Ser Val Ala Glu Gly Val Lys Ala Gly Ile Gln Glu Cys Gln His Gln
65                  70                  75                  80
Phe Arg Gly Arg Arg Trp Asn Cys Thr Thr Val Ser Asn Ser Leu Ala
                85                  90                  95
Ile Phe Gly Pro Val Leu Asp Lys Ala Thr Arg Glu Ser Ala Phe Val
            100                 105                 110
His Ala Ile Ala Ser Ala Gly Val Ala Phe Ala Val Thr Arg Ser Cys
        115                 120                 125
Ala Glu Gly Ser Ala Ala Ile Cys Gly Cys Ser Ser Arg Leu Gln Gly
    130                 135                 140
Ser Pro Gly Glu Gly Trp Lys Trp Gly Gly Cys Ser Glu Asp Ile Glu
145                 150                 155                 160
Phe Gly Gly Met Val Ser Arg Glu Phe Ala Asp Ala Arg Glu Asn Arg
                165                 170                 175
Pro Asp Ala Arg Ser Ala Met Asn Arg His Asn Asn Glu Ala Gly Arg
            180                 185                 190
Gln Ala Ile Ala Ser His Met His Leu Lys Cys Lys Cys His Gly Leu
        195                 200                 205
Ser Gly Ser Cys Glu Val Lys Thr Cys Trp Trp Ser Gln Pro Asp Phe
    210                 215                 220
Arg Thr Ile Gly Asp Phe Leu Lys Asp Lys Tyr Asp Ser Ala Ser Glu
225                 230                 235                 240
Met Val Val Glu Lys His Arg Glu Ser Arg Gly Trp Val Glu Thr Leu
                245                 250                 255
Arg Pro Arg Tyr Thr Tyr Phe Lys Val Pro Thr Glu Arg Asp Leu Val
            260                 265                 270
Tyr Tyr Glu Ala Ser Pro Asn Phe Cys Glu Pro Asn Pro Glu Thr Gly
        275                 280                 285
Ser Phe Gly Thr Arg Asp Arg Thr Cys Asn Val Ser Ser His Gly Ile
    290                 295                 300
Asp Gly Cys Asp Leu Leu Cys Cys Gly Arg Gly His Asn Ala Arg Thr

305                 310                 315                 320
Glu Arg Arg Arg Glu Lys Cys His Cys Val Phe His Trp Cys Cys Tyr
                325                 330                 335
Val Ser Cys Gln Glu Cys Thr Arg Val Tyr Asp Val His Thr Cys Lys
            340                 345                 350